# EUROPEAN PATENT APPLICATION

(11) **EP 4 552 630 A1**
(43) Date of publication of application: **14.05.2025**
(21) Application number: 23209390.6
(22) Date of filing: 13.11.2023
(51) Int. Cl.: A61K 8/41, A61K 8/49, A61Q 5/10

(54) **RESORCINOL FREE PERMANENT HAIR COLOR COMPOSITION AND METHOD**

(71) Applicant: Wella Operations US, LLC, Calabasas, CA 91302 (US)
(72) Inventor: VOHRA, Firoj, New Jersey (US)
(74) Representative: Dennemeyer & Associates S.A.

(57) **Abstract**

The present invention is directed to a resorcinol free oxidative hair coloring composition based upon the dye compounds 2-amino-5-ethyl phenol, hydroxyethyl-3,4-methylenedioxyaniline and 2-methoxymethyl-p-phenylene diamine. Additional precursors and couplers may be included to broaden the color space delivered by the primary dye compounds. The color space of the resorcinol free oxidative hair coloring composition as measured by L*a*b* techniques is the same as that delivered by the resorcinol based composition.

## Description

### Field of the Invention

The invention is directed to an oxidative hair coloring composition and method for its use on anagenic hair. The oxidative hair coloring composition is based upon a combination of oxidative couplers and oxidative primary dye precursors that deliver a brown to gold color space for the treated hair, roots to tips, that is substantially to essentially similar to the color space delivered by a resorcinol based hair coloring composition. The oxidative hair coloring composition according to the invention does not include resorcinol.

### Background of the Invention

The hair coloring technique utilizing permanent oxidative color compositions is well-known in the art. Permanent and demi-permanent oxidative hair coloring products have been used in professional salons and in retail products for use at home for decades. Coloring products typically comprise a tint composition and an oxidizing composition which are packaged separately and mixed immediately before use to form the coloring composition. The tint composition typically contains one or more primary oxidative dye precursors, one or more oxidative couplers and an alkalizing agent, usually ammonia or an amine such as mono-ethanol amine. The primary oxidative dye precursors react with the oxidative couplers in the presence of an oxidizing agent to form larger, colored dye products within the hair. The oxidizing agent, typically hydrogen peroxide, enables the coloring process through its oxidation of the primary oxidative dye precursor(s) which react with the coupler(s) to form the large colored dye products. The hydrogen peroxide, especially in the presence of an alkalizing agent, also bleaches the natural melanin of the hair, so that the original hair color is minimized or eliminated and new shades of hair color lighter or darker than the original shade can be produced. This process is described for example in the European Scientific Committee on Consumer Products Opinion 1198/08, January 2009.

To form the oxidative coloring composition, the tint composition and oxidizing composition are mixed together immediately prior to the application to the hair to be colored. The resulting oxidative coloring composition usually has an alkaline pH between about 8.5 and 10.5 and is applied and left on the hair usually for 5 to 50 minutes before rinsing.

According to the state of the art, creation of a large variety of different shades generally involves a combination of different oxidative coupler dyes precursors and oxidative primary dye precursors. Traditionally, the couplers have been based upon resorcinol. This coupler has been able to produce rich color spaces spanning the visible color spectrum when combined with selected variations of the oxidative dye precursors. However, recent safety, health and environmental issues have been raised with respect to the use of resorcinol. Questions regarding allergic reactions, sensitizing potency and long term toxic and cellular alteration effects have been raised with the effect that governmental regulatory agencies have begun considering a ban on the use of the resorcinol coupler.

Aside from the health, environmental and safety issues an additional issue with such permanent hair colorants is that a uniform degree of permanent coloration along the hair strands from root to tip is difficult to achieve. Typically, the concentration of the oxidative hair composition is varied as it is applied to these different regions of the hair strands. Moreover, because hair continues to grow from the scalp, a person must recolor the hair, especially the hair roots where natural color reappears. When the re-coloration is done, the mid-lengths and tips of the hair strands become ever darker but the roots only receive the color of the re-coloration. This leads to an uneven appearance of the hair coloration.

While resorcinol based oxidative hair compositions have been able to manage root to tip coverage and provide an even appearance, non-resorcinol based oxidative hair compositions have been found to be unable to achieve similar results even when their color space spectra mimic those at produced by resorcinol based compositions. Experimentation with mixtures of precursors and supplemental couplers has been unable to manage root to tip coverage. This unfortunate result typically occurs when the color space of the non-resorcinol oxidative hair composition is similar to that of the resorcinol based oxidative hair composition.

There is consequently a need for providing oxidative hair coloring compositions that do not utilize resorcinol, deliver color space spectra similar to those of resorcinol based oxidative hair coloring compositions and enable at least substantially uniform root to tip coverage. A further need is to provide coupler replacements that are regarded as having lower and/or no concerns in regard to safety, environmental, health and toxicity issues. At the same time there is a need to provide oxidative hair coloring compositions that mimic the rich color spaces over the entire visible light spectrum provided by the resorcinol oxidative hair dye technology.

### SUMMARY OF THE INVENTION

These and other needs are achieved by the present invention which is directed to an artistically designed oxidative coloration technology. The oxidative coloration technology is free of resorcinol yet provides a brown to gold visible color space substantially to essentially similar to the brown to gold visible color space produced by resorcinol hair coloring compositions. Moreover, choice of the chassis carrying the non-resorcinol oxidative coloration technology has been found to enable substantially uniform root to tip coverage.

A first aspect of this technology includes embodiments of an oxidative hair coloring composition incorporating at least one or more oxidative dye precursor components and one or more oxidative coupler components as well as the chassis composition which together constitute the tint composition part of the oxidative hair coloring composition. Additional aspects of this technology include embodiments of a kit incorporating a tint composition including the one or more oxidative dye precursor and oxidative coupler components and a separate oxidizer component. A still further aspect includes embodiments of a method for forming the oxidative hair coloring composition from the kit and applying the oxidative hair coloring composition to the hair.

Embodiments of the oxidative hair coloring composition are directed to a combination of at least one or more oxidative dye precursors and one or more oxidative couplers in a cosmetically acceptable aqueous or aqueous-organic medium. These embodiments constitute the tint composition of the oxidative hair coloring composition.

Accordingly, embodiments of the tint composition for the oxidative hair coloring composition comprise at least components A, B and C:
A) at least a first oxidative coupler comprising 2-amino-5-ethylphenol of Formula I, its addition salts, its solvates and/or solvates of its salts, 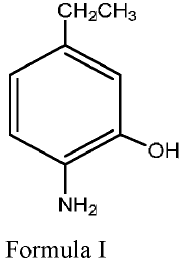
B) at least a second oxidative coupler comprising hydroxylethyl-3,4-methylenedioxyaniline of Formula II, its addition salts, it solvate and/or solvates of its salts, 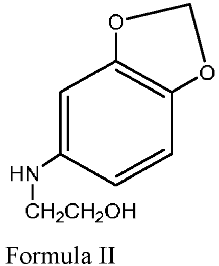
C) at least one oxidative primary dye precursor comprising 2-methoxymethyl-p-phenylene diamine of Formula III, its addition salts, its solvates and/or solvates of its salts.

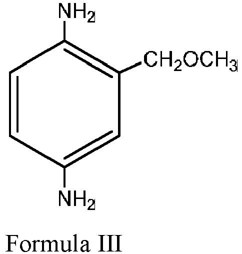

Additional embodiments of the oxidative hair coloring composition of at least components A, B and C according to the invention further comprise component D, which is at least one or more additional oxidative dye precursors and component E, which is at least one or more additional oxidative couplers.
Component D comprises at least one or more of:
   i) N,N-bis(2-hydroxyethyl)-p-phenylenediamine, its addition salts, its solvates and/or solvates of is salts; and/or,
   ii) p-aminophenol, its addition salts, its solvates and/or solvates of is salts; and/or,
   iii) any combination of Di) and ii).
Component E comprises at least one or more of:
   i) 2,4-diaminophenoxyethanol, its addition salts, its solvates and/or solvates of its salts; and/or,
   ii) m-aminophenol, its addition salts, its solvates and/or solvates of its salts; and/or,
   iii) 2-methyl-5-hydroxyethylaminophenol, its addition salts, its solvates and/or solvates of its salts; and/or,
   iv) 1-naphthol and/or its solvates; and/or,
   v) 1-phenyl-3-methyl-5-pyrazolone, its addition salts, its solvates and/or solvates of its salts; and/or,
   vi) any combination of E i), ii), iii) iv) and v).
Included as well is any combination of components Di), ii), iii), Ei), ii), iii), iv), v) and vi).

Yet another embodiment of the oxidative hair coloring composition according to the invention provides that component C as 2-methoxymethyl-p-phenylene diamine is replaced by 2,5-toluenediamine sulfate as component C.

A second aspect of the tint composition part of this technology includes embodiments that incorporate choice of the chassis composition for delivery of substantially uniform root to tip coverage. Embodiments of the chassis include two kinds of surfactant: a phosphate ester/acid surfactant/fatty alcohol combination and a nonionic surfactant with viscosity control agent and no fatty acid or fatty ester. Additional oxidative hair coloring ingredients include alcohol solvent, water, an alkalizer and a chelant. More specifically, the chassis embodiments include the dual phosphate surfactants dicetyl phosphate and ceteth-10 phosphate in combination with cetearyl alcohol, the non-ionic surfactant steareth-200, xanthan gum viscosity control, alkalizers including ammonium hydroxide, aminopropanol, ethanol amine, ammonium carbonate, and chelants trisodium ethylenediamine disuccinate and sodium diethylentriamine pentamethylene phosphonate.

The embodiments of the oxidative hair coloring composition according to the invention may additionally include one or more supplemental oxidative couplers and one or more supplemental oxidative dye precursors as well as ancillary chassis ingredients including one or more anionic surfactants, one or more nonionic surfactants, one or more fatty substances, one or more conditioners, one or more viscosity control agents, one or more humectant/emulsifiers, one or more alkalizers, one or more antioxidants, one or more pH control agents, one or more chelates and a cosmetically acceptable aqueous or aqueous organic medium.

The embodiments of the oxidative hair coloring composition according to the invention may further include an oxidizer composition part of the oxidative hair coloring composition such as hydrogen peroxide to facilitate the oxidative coupling of the oxidative dye precursor(s) and oxidative coupler(s).

Another aspect of the invention concerns a kit of the components that can be combined to form the oxidative hair coloring composition described above. The kit comprises a tint composition of at least one or more of the above described oxidative dye precursor components C and D, and at least one or more of the above described oxidative coupler components A, B and E. The kit also separately comprises an oxidizer composition of at least hydrogen peroxide.

A further aspect of the invention concerns a method for coloring hair using the above described kit. The tint composition and the oxidizer composition of the kit are combined to form the oxidative hair coloring composition immediately before its application to the hair. The resulting oxidative hair coloring composition is applied to the hair and allowed to remain on the hair for a period of from about 5 minutes to about 50 minutes. Following this application period, the hair is rinsed with water to remove the oxidative hair coloring composition.

The method for coloring hair with the oxidative hair coloring composition produced by combining the tint and oxidizer compositions of the kit produces hair having a brown and gold tone and hue showing a visible color space spectrum throughout roots and tips at least very similar to the visible color space spectrum of the colors produced on hair by a resorcinol oxidative hair dye composition touted as giving brown and gold tone and hue as demonstrated by the examples below.

### DEFINITIONS

Unless defined otherwise, all technical and scientific terms used herein have the same meaning as commonly understood by a person of ordinary skill in the art.

As used in the specification and the appended statements, the singular forms "a," "an" and "the" include plural referents unless the context clearly dictates otherwise. Also use of a plural term describing a thing or element includes the singular unless the context clearly dictates otherwise. For example, the terms fatty alcohols or hydrocarbons include a single fatty alcohol or a single hydrocarbon as well as multiples.

The term "may" in the context of this application means "is permitted to" or "is able to" and is a synonym for the term "can." The term "may" as used herein does not mean possibility or chance.

The term and/or in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4. Similarly, values expressed in a range format should be interpreted in a flexible manner to include not only the numerical values explicitly recited as the limits of the range, but also to include all the individual numerical values or sub-ranges encompassed within that range as if each numerical value and sub-range were explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range.

The terms "comprising", "comprises" and "comprised of' as used herein are synonymous with "including", "includes" or "containing", "contains", and are inclusive or open-ended and do not exclude additional, non-recited members, elements or method steps.

The term "anagenic hair" as used herein means hair strands that are in direct connection with a hair follicle which is in either the anagen or telogen state. Anagenic hair is present in one of these states on a scalp of a person, a human, and its follicle is appurtenant to a sebum gland that substantially continuously secretes sebum and long chain fatty acids onto the surfaces of the hair shaft. As the hair grows from the follicle, it carries with it coatings of secreted sebum and long chain fatty acids. The fatty acid coating is known as the f layer and is tightly entwined with the keratin protein at the hair shaft surface. Hair cut from a living person is no longer anagenic hair.

Keratin fibers means hair strands on the scalp of a person, preferably anagenic hair. Keratin fibers, aka hair strands, are comprised of cells forming an inner cortex as the core and a cuticle as the outer covering surrounding the cuticle. The hair strand chemical components include proteins, poly-nucleic acids, amino acids, minerals, melanin, lipids including fatty acids, fatty alcohols, triglycerides, phospholipids, cholesterol, and squalene. These components are configured as cells, fibrils, connective tissue, extracellular links, pigments, keratin and associated structures. The outer layer of the cuticle is configured as a sheath of overlapping flat cells having a structure like the bark of a palm tree or scales of a fish. Like the scales of a fish, the surface of the cuticle is smooth in the direction from the root to the tip and rough in the direction from the tip to the root.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "preferred embodiment" means that a particular feature, structure, or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment but may refer to different embodiments of the presently claimed invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

For the purposes of the presently claimed invention, `% by weight' or 'wt.% `as used in the presently claimed invention is with respect to the total weight of the oxidative hair coloring composition. Further, the sum of wt.% of all the compounds (components), as described herein, in the respective compositions add up to 100 wt.%.

The term "oxidative hair coloring composition", means a ready-to-use composition with and without oxidizer. With oxidizer, the oxidative hair coloring composition is ready to change the color of hair on which it is applied. Embodiments and aspects of the oxidative hair coloring composition are described in the foregoing Summary and in the following Detailed Description.

The term "two-component" oxidative hair coloring system means the two separate components including a tint composition and an oxidizing composition that may be combined to form the above-described oxidative hair coloring composition. The tint composition may but not necessarily be the same as the oxidative hair coloring composition without oxidizer. In some situations, chassis components of the oxidative hair coloring composition may have come only from the oxidizer or oxidizing composition. In particular, an acid such as citric acid for maintaining the pH of the oxidizer composition at an acidic pH may not be present in the tint composition because the pH of the tint composition is maintained at a basic pH.

The term "user" means the person preparing the hair coloring composition. The user may be, for example, a professional hair stylist working in a salon and is different from the subject on whose hair the composition is applied. The user, for example, may also be identical to the person on whose hair the composition is applied.

The term "resorcinol" means 1,3-dihydroxybenzene as applied to cosmetic oxidative hair dye systems.

The term "color space" means the visible light spectrum measured by well-known parameters such L*a*b* values or CIELAB color space. The Wikipedia explanation of the CIELAB color space is the understanding of this term applied here (Wikipedia, "CIELAB color space", August 2, 2023). CIELAB color space, also referred to as L*a*b*, is a color space defined by the International Commission on Illumination (abbreviated CIE) in 1976. It expresses color as three values: L* for perceptual lightness and a* and b* for the four unique colors of human vision: red, green, blue and yellow. CIELAB was intended as a perceptually uniform space, where a given numerical change corresponds to a similar perceived change in color. While the LAB space is not truly perceptually uniform, it nevertheless is useful in industry for detecting small differences in color.

The CIELAB color space is a device-independent, "standard observer" model. The colors it defines are not relative to any particular device such as a computer monitor or a printer, but instead relate to the CIE standard observer which is an averaging of the results of color matching experiments under laboratory conditions.

The CIELAB space is three-dimensional and covers the entire gamut (range) of human color perception. It is based on the opponent color model of human vision, where red and green form an opponent pair and blue and yellow form an opponent pair. The lightness value, *L**, also referred to as "Lstar," defines black at 0 and white at 100. The *a** axis is relative to the green-magenta opponent colors, with negative values toward green and positive values toward magenta. The *b** axis represents the blue-yellow opponents, with negative numbers toward blue and positive toward yellow.

### DETAILED DESCRIPTION OF THE INVENTION

The present invention is directed to an oxidative hair coloring composition composed of two combinable and/or combined components or parts, a tint composition and an oxidizer composition, the corresponding two component oxidative hair coloring system characterized herein as a kit, and a method of applying the oxidative hair coloring composition to anagenic hair.

The aspect of the invention concerning the oxidative hair coloring composition involves a combination of oxidative couplers and oxidative primary dye precursors that deliver an L*a*b* color space substantially or essentially like the L*a*b* color space delivered by resorcinol based dye compositions but the oxidative hair coloring composition according to the invention does not include resorcinol.

Embodiments of the oxidative hair coloring composition and its tint composition part include at least a combination of components A and B which respectively are 2-amino-5-ethyl phenol and hydroxyethyl-3,4-methlenedioxyaniline (acronyms E-OAP and Aminol respectively) as oxidative couplers and component C which is 2-methoxymethyl-p-phenylene diamine (acronym MBB) as an oxidative primary dye precursor. The forms of components A, B and C include their non-neutralized amines as well as their addition salts, solvates and solvates of these salts.

An alternative embodiment of the oxidative hair coloring composition and its tint composition including at least a combination of components A, B and C comprises replacement of 2-methoxymethyl-p-phenylene diamine as component C by 2,5-toluenediamine sulfate as component C. The following embodiments of the oxidative hair coloring composition as well as all other component descriptions such as but not limited to chassis components include component C either as 2-methoxymethyl-p-phenylene diamine or as 2,5-tolueneniamine sulfate.

Embodiments of the oxidative hair coloring composition and its tint composition comprising components A, B and C may further comprise include at least one additional oxidative primary dye precursor, component D, and/or at least one oxidative coupler component E. Component D may be at least one of Di) N,N-bis(2-hydroxyethyl-p-phenylenediamine and/or Dii) p-aminophenol as well as the addition salts, solvates and/or solvates of the salts of Di) and Dii). Included as well is Diii) which is any combination of Di) and Dii and any of their addition salts, solvates and/or solvates of their salts. Component E may be at least one of Ei) 2,4-diaminophenoxyethanol, Eii) m-aminophenol, Eiii) 2-methyl-5-hydroxyethylaminophenol, Eiv)1-naphthol and/or Ev) 1-phenyl-3-methyl-5-pyrazolone as well as the addition salts, solvates and solvate of the salts as applicable for Ei) -Ev). Included as well is Evi) which is any combination of Ei) - Ev) and any of their addition salts, solvates and/or solvates of their salts. Additional combinations include any combination of Di), Dii), Ei)-Ev), Diii) and/or Evi).

The combinations of components A-E and their weight percentages or concentrations in the oxidative hair coloring composition are made according to the experienced dye color designer's understanding of the nuances in tint, shade and tone that each oxidative primary dye precursor and each oxidative coupler added to the oxidative coloring composition delivers to the tint, shade and tone providing the desired L*a*b* color space. Depending upon the character of the natural color of the anagenic hair to be colored, the concentrations of components A-E may be varied and some or all of the members of components D and E may be included. In general to achieve an L*a*b* color space substantially or essentially like that achieved by a resorcinol based oxidative dye system, a weight percent ratio of the oxidative couplers, components A, B to the oxidative dye precursor component C may range from 1: 1 to 1:1.5. When the additional components Di), Dii), Ei) - Ev) are included, the weight percent ratio of oxidative couplers, [weight percentage sum of A, B, and one or more of Ei) -Ev)] to the oxidative dye precursors [weight percentage sum of C and one or both of Di) and Dii)] may be approximately the same weight ratio range of 1: 1 to 1:1.5. The concentrations of each of the oxidative dye precursors and oxidative couplers may in a weight percent range of from about 0.1 wt% to about 10 wt%, preferably 0,1 wt% to about 5 wt% with the sum of the concentrations of all precursors and couplers being from 0.5 wt % to about 10 wt%. The demonstration that the L*a*b* color space of the oxidative hair coloring composition is essentially the same as the L*a*b* color space of the composition with resorcinol substituted for component A is shown by the experimental results given below.

### Additional oxidative couplers and oxidative primary dye precursors

Embodiments of the oxidative hair coloring composition and its tint composition of at least components A, B, C, D and E may additionally comprise component F, at least one additional oxidative coupler and/or component G, at least one additional oxidative primary dye precursor.

### Component (F)

The oxidative hair coloring composition and its tint composition may include one or more additional oxidative couplers depending upon the color spectrum to be achieved. The one or more additional oxidative couplers denoted herein as component F may increase the total concentration of all couplers present or may be a substitute for a portion of the other couplers already present. The color affect or change such additional couplers have in combination with the oxidative dye precursors present in the oxidative hair coloring composition is well-known and is accounted according to the desired color outcome and color space desired. Inclusion of component F in the oxidative hair coloring composition may be accomplished by addition of one additional oxidative coupler and/or by a mixture of two or more different further oxidative couplers.

In an embodiment of the presently claimed invention, the oxidative hair coloring composition and its tint composition may additionally comprise as component F at least one additional oxidative coupler selected from the group consisting of m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethyl-aminotoluene, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindoline, phenyl methyl pyrazolone, 5-((2-hydroxyethyl)amino)-1,3-benzodioxol, hydroquinone, 4-formyl-1-methylquinolinium-p-toluenesulfonate and addition salts, solvates and/or solvates of salts thereof.

### Component (G)

In one embodiment the oxidative hair coloring composition and its tint composition may additionally comprise as component (H) at least one additional oxidative primary dye precursor. The one or more additional oxidative primary dye precursors denoted herein as component G may increase the total concentration of all primary dye precursors present or may be a substitute for a portion of the other primary dye precursors already present. The color affect or change such additional primary dye precursors have in combination with the oxidative couplers is well-known and is accounted according to the desired color outcome and color space desired. Inclusion of component G in the oxidative hair coloring composition may be accomplished by addition of one additional oxidative primary dye precursor and/or by a mixture of two or more different further oxidative primary dye precursors.

In one embodiment of the presently claimed invention, the oxidative hair coloring composition and its tint composition additionally comprises as component G at least one additional oxidative primary dye precursor selected form the group consisting of hydroxyethyl-p-phenylenediamine sulphate, hydroxypropyl bis(n-hydroxyethyl-p-phenylenediamine), 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and addition salts, solvates and/or solvates of salts thereof.

### Medium

The medium of the oxidative hair coloring composition and the kit of the tint composition and the oxidizer composition may be a "cosmetically acceptable aqueous or aqueous-organic medium." Exemplary media include deionized water alone and deionized water in combination with a liquid organic solvent such as one or more of a C1-C4 monoalcohol, a C2-C6 diol, a C3-C6 triol, a C3-C4 ketone and similar organic solvents typically used as cosmetically acceptable media provided that such organic solvents are not chemically reactive with any of the components of the oxidative hair coloring composition. Typical organic solvents for mixture with water to form a cosmetically acceptable aqueous-organic medium include ethanol, isopropanol, n-propanol, butanol, glycerin, ethylene or propylene glycol, and hexane glycol.

The medium concentration for the oxidative hair coloring composition may be in a range of ≥ 30.0 to ≤ 95.0 wt.%, preferably in an amount in the range of ≥ 35.0 to ≤ 92.0 wt.%, more preferably in an amount in the range of ≥ 40.0 to ≤ 90.0 wt.%, based on the total weight of the oxidative hair coloring composition. The concentration of organic solvent in water for the aqueous-organic medium may be in a range of from about 1% to about 50%, preferably about 1% to about 25% by volume relative to the total volume of the medium.

### Oxidizing agent. Component H

The formation of the color in the anagenic hair is accomplished by oxidation of the oxidative dye precursor or precursors and the reaction with the oxidative coupler or couplers to form large dye molecules inside the hair strands. The oxidizing agent (Component H) typically included with the oxidative hair coloring composition is hydrogen peroxide although other oxidizing agents can serve this function.

Because this oxidative reaction takes place upon the combination of the oxidizing agent and the oxidative dye precursors and oxidative couplers, the oxidizing agent is typically maintained in a separate composition until the oxidative hair coloring composition is to be applied to anagenic hair. The separate composition is characterized herein by the description of the kit. The kit comprises the tint composition of at least components A-C with optional components D-G and I (alkalizer) and the separate oxidizer composition of at least component H.

Typically, the oxidizing agent as the oxidizer composition of the kit is combined with the tint composition of the kit containing other components of the oxidative hair coloring composition immediately before its application to anagenic hair. Under ordinary conditions, the oxidizer composition as a separate composition includes ancillary carrier ingredients such as a fatty substance, a surfactant, a hydrocarbon and an aqueous or organo-aqueous medium. The pH of the oxidizer composition is rendered acidic by inclusion of cosmetically acceptable organic and/or inorganic acid, such as citric acid, etidronic acid and the like. When the oxidizer composition is combined with the tint composition, e.g., the oxidative hair coloring composition without oxidizing agent, the alkalizing agent of the tint composition changes the pH of the resulting mixture to basic. The oxidizing ability of the hydrogen peroxide is better at a basic pH relative to its ability at an acidic pH, For this reason, the term "oxidative hair coloring composition" includes this composition with and without the oxidizing agent.

The oxidizing agent may be a water-soluble inorganic peroxide material which is capable of yielding hydrogen peroxide in an aqueous solution. In one embodiment, the at least one oxidizing agent may be chosen from such compounds as hydrogen peroxide, inorganic alkali metal peroxides (such as sodium periodate and sodium peroxide), organic peroxides (such as urea peroxide, melamine peroxide), inorganic perhydrate salt bleaching compounds (such as the alkali metal salts of perborates, percarbonates, perphosphates, persilicates, persulphates), and mixtures thereof; preferably from the group consisting of hydrogen peroxide, persulphates, and mixtures thereof. Most preferably the oxidizing agent is hydrogen peroxide.

The term "water-soluble," in this context, means that in standard condition at least 0.1 g, 1 g, or 10 g of the oxidizing agent can be dissolved in 1 liter of deionized water.

In one embodiment, the component H in the oxidative hair coloring composition with oxidizer is preferably present in an amount in the range of ≥ 1.0 wt.% to ≤ 8.0 wt.%, more preferably in an amount in the range from of ≥ 1.0 wt.% to ≤ 6.0 wt.%, more preferably in an amount in the range from of ≥ 1.2 wt.% to ≤ 5.0 wt.%, based on the total weight of the oxidative hair coloring composition. Because the oxidizing agent or oxidizer is maintained in as a separate composition until the oxidative hair coloring composition is to be applied to hair, as explained above, the concentration of component H, the oxidizing agent, in the separate oxidizer composition of the kit is determined by back calculation from its final concentration to be used in the oxidative hair coloring composition.

The oxidizing agent can be provided in aqueous solution or as a powder which is dissolved prior to use.

### Chassis

The embodiments of the oxidative hair coloring composition also may optionally but not necessarily include one or more ancillary cosmetic carrier ingredients also known as chassis ingredients. The chassis ingredients include but not limited to, one or more alkalizers (component I) one or more anionic and/or nonionic surfactants (component J), one or more fatty substances (component K), one or more chelators (component L), one or more viscosity control agents (component M), one or more conditioners (component N), one or more humectants/emulsifiers, one or more antioxidants and one or more pH control agents as well as other usual and typical ancillary ingredients well-known for use with for oxidative hair dye technology.

### Alkalizer, Component I

The alkalizer or alkalizing agent of the oxidative hair coloring composition may be present in the tint composition carrying the dye components and ancillary carriers and not in the oxidizer composition (e.g., the tint composition of the kit described above).

Embodiments of the oxidative hair coloring composition may include component I, the alkalizer or alkalizing agent, as a C2-C4 organic amine, a C2-C5 organic alcohol amine or as ammonia or ammonium hydroxide or ammonium carbonate. A preferred alkalizing agent may be 2-amino-1-propanol, 2-amino-1-butanol, 2-dimethylamino-2-pethyl-1-propanol, 2-amino-2-methylpropan-1-ol, 3-amino-1-propanol, ammonia, or monoethanolamine. An especially preferred alkalizing agent is ammonia, 2-amino-1-propanol, ammonium carbonate and/or monoethanolamine.

The alkalizer, component I, may be present in the oxidative hair coloring composition in an amount in the range of ≥ 0.1 wt.% to ≤ 10.0 wt.%, preferably in an amount in the range of ≥ 0.1 wt.% to ≤ 7.0 wt.%, particular preferred in an amount in the range of ≥ 1.0 wt.% to ≤ 6.3 wt.%, and most preferred in an amount in the range of ≥ 1.0 wt.% to ≤ 2.5 wt.%, in each case based on the total weight of the oxidative hair coloring composition.

When the oxidative hair coloring composition is formulated as a kit comprising the tint composition and the oxidizer composition as described above, the alkalizer typically is present in the tint composition. When the tint composition is combined with the oxidizer composition, the concentrations of the components of the tint composition are diluted according to the volume or weight ratio of the tint composition to the oxidizer composition. Accordingly, the concentration of the alkalizer, component I, needed in the tint composition may be calculated from the desired final concentration in the oxidative hair coloring composition and the dilution ratio of the tint and oxidizer compositions.

### Component J - Anionic, Cationic and/or Nonionic Surfactant

The anionic, cationic and/or nonionic surfactant provides a solubilizing function for components of the oxidative hair coloring composition as well as the kit constituents, the tint and oxidizer compositions that are insoluble, slightly soluble and moderately soluble in water. The anionic, cationic and/or nonionic surfactants also are believed to participate in the transport of the components of the oxidative hair coloring composition onto and into the strand of anagenic hair at least in part by mingling and/or interacting with the sebum, f layer and strand surface compounds present on anagenic hair.

The anionic surfactant spans the categories of phosphate anionic surfactant, sulfate anionic surfactant and carboxyl anionic surfactant such as but not limited to one or more alkyl and/or polyoxyalkyl alkyl phosphates and/or one or more alkyl sulfates and/or polyoxyalkylene alkyl sulfates and/or one or more alkyl carboxylates and/or polyoxyalkylene alkyl carboxylates.

The nonionic surfactant spans the categories of polyethoxyalkylether nonionic surfactant, alkamido betaine zwitterionic surfactant, polysorbate surfactant, glucoside and polyglucoside nonionic surfactant, fatty alkyl pyrrolidone nonionic surfactant, polyoxyalkylene fatty acid ester nonionic surfactant, alkylpolyoxyalkylenalkanamidoalkyl alcohol nonionic surfactant.

The cationic surfactant spans the categories of higher alkyl di and/or trimethyl ammonium cationic surfactant, poly(dimethyliminoalkylenyl) alkane cationic surfactant,
Representative examples of anionic surfactants include salts (such as alkaline salts, for example, sodium salts, ammonium salts, amine salts, amino alcohol salts and magnesium salts) of the following compounds: alkyl phosphates, alkyl ether phosphates, C8 to C30 alkyl phosphate, C8 to C30 alkyl polyoxyalkyl-alkyl ether phosphate or mixtures thereof, alkyl ether sulphates, alkyl glyceryl sulphonates, alkylamido ether sulphates, alkylarylpolyether sulphates, alkyl monoglyceride sulphates, alkyl ether sulphonates, alkylamide sulphonates; alkyl succinates, alkyl sulphosuccinates, alkyl ether sulphosuccinates, alkylamide sulphosuccinates; alkyl sulphosuccinamates; alkyl sulphoacetates; alkyl ether phosphates; acyl sarcosinates, N-acyl methylaminopropionate; acyl isethionates, N-acyl taurates; acyl lactylates; carboxyalkyl ether of alkyl polyglucosides; alkyl lecithin derivatives. The alkyl or acyl radical of all of these various compounds, for example, comprises from about 8 to 30 carbon atoms, and the aryl radical, for example, is chosen from phenyl and benzyl groups.

Preferred exemplary anionic surfactants include alkyl phosphates, polyoxyalkyl-alkyl ether phosphates, alkyl ether sulphates, alkyl glyceryl sulphonates, N-acyl sarcosinates, N-acyl taurates, acyl lactylates and carboxyalkyl ether of alkyl polyglucosides. Yet, more preferable surfactants are selected from alkyl phosphates and polyoxyalkyl-alkyl ether phosphates having an average 1 to 20, 1 to 10 or 1 to 3 ethylene oxide units.

More preferred phosphate anionic surfactants include dicetyl phosphate, ceteth-10 phosphate alone or together or in combination with cetearyl alcohol having the trade name Crodafos sold by Croda International, Snaith, United Kingdom.

More preferred sulfate anionic surfactants include laureth-2 to 200 sulfate, cocyl isethionate and lauryl sulfate.

Representative examples of the nonionic surfactants include polyoxyalkylene-fatty alcohol esters of fatty acids, alkyl polyoxyalkylene ethers which are derived from C1-C6-alcohols or from C7-C30-fatty alcohols, alkylaryl alcohol polyoxyethylene ethers, alkoxylated animal and/or plant fats and/or oils, glycerol esters, alkylphenol alkoxylates, fatty amine alkoxylates, fatty acid amide and fatty acid diethanolamide alkoxylates, ethoxylates thereof, sugar surfactants, sorbitol esters, polyoxyethylene sorbitan fatty acid esters, alkyl polyglycosides, N-alkylgluconamides and alkyl methyl sulfoxides.

Preferred exemplary nonionic surfactants include steareth-2 to 200, octadeceth-2 to 200, laureth-2 to 200, undeceth-2 to 200, pareth-2-200, cocamidopropyl betaine, polysorbate -2 to 80 polyoxyethylene, lauryl pyrrolidone and any combination thereof. More preferred exemplary nonionic surfactants include steareth 2 to 200, octadeceth 2-200 (also known as Brij 2-200) and pareth 2-200 and any combination thereof.

The cationic surfactant useful as a surfactant with the oxidative hair coloring composition may be one or more quaternary ammonium salts or amido-amines having at least one fatty chain containing at least about 20 carbon atoms and mixture thereof.

Generally, the quaternary ammonium salts useful as cationic surfactants for the oxidative hair coloring composition follow the general formula N⁺(R¹R²R³R⁴)X⁻: wherein, R¹ is selected from linear and branched radicals comprising about 20 to 30 carbon atoms, R² is selected from linear and branched radicals comprising about 20 to 30 carbon atoms or the same group as radicals R³ to R⁴, the radicals R³ to R⁴, which can be identical or different, are selected from linear and branched aliphatic radicals comprising from about 1 to 4 carbon atoms, and aromatic radicals such as aryl and alkylaryl, the aliphatic radicals can comprise at least one hetero atom such as oxygen, nitrogen, sulphur and halogens, the aliphatic radicals are chosen, for example, from alkyl, alkoxy and alkylamide radicals, and wherein X⁻ is an anion selected from halides, such as chloride, bromide and iodide) (C₂-C₆)alkyl sulphates, such as methyl sulphate, phosphates, alkyl and alkylaryl sulphonates, and anions derived from organic acids, such as acetate and lactate.

Representative examples of a cationic surfactant for use with the oxidative hair coloring composition include behentrimonium chloride, behenamidopropyltrimonium methosulfate, stearamidopropyltrimonium chloride, arachidtrimonium chloride and mixtures thereof.

Generally, the amido-amine cationic surfactants follow the general formula R'¹-CONH(CH₂)ₙNR'²R'³: wherein, R'¹ is selected from linear and branched radicals comprising about 20 to 30 carbon atoms, the radicals R'² and R'³, which can be identical or different, are selected from hydrogen, linear and branched aliphatic radicals comprising from about 1 to 4 carbon atoms, and aromatic radicals such as aryl and alkylaryl, the aliphatic radicals can comprise at least one hetero atom such as oxygen, nitrogen, sulphur and halogens, the aliphatic radicals are chosen, for example, from alkyl, alkoxy and alkylamide radicals, and wherein n is integer from 1 to 4.

Representative examples of the amido-amine nonionic surfactant include behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine, arachidamidoethyidimethylamine, and mixtures thereof.

Preferably, the surfactant, component J, is an anionic surfactant or non-ionic or a combination of both. More preferably the surfactant component J is a combination of dicetyl phosphate, ceteth-10 phosphate together with cetearyl alcohol optionally with a non-ionic surfactant such as cetereth-2-200, brij-2-200 and/or pareth-2-200. Alternatively and more preferably, the surfactant component J is laureth sulfate or lauryl sulfate optionally in combination with a non-ionic surfactant such as seteareth-2-200, brij-2-200 and/or pareth-2-200 or any combination thereof. Alternatively and preferably, the surfactant component J is seteareth-2-200, brij-2-200, pareth-2-200 and/or cocoamidopropyl betaine and/or any combination thereof.

### Component K, Fatty Substance

The fatty substance component K includes lipophilic/ hydrophobic substances that are insoluble or slightly soluble in water. The fatty substances are soluble in organic solvents such as chloroform, ethanol, or benzene. The fatty substances may be liquids or solids, usually low melting solids as STP. The fatty substances include the categories of C8-C36 alkyl and alkenyl alcohols, C8-C36 alkyl and alkenyl carboxylic acids, Esters of C8-C36 alkyl and alkenyl carboxylic acid with C2-C6 mono, di and/or tri alkanols, and C10 -C36 linear and branched saturated and unsaturated hydrocarbons. One, several and/all of these categories of fatty substance may be included in the oxidative hair coloring composition. The fatty substances provide hair conditioning effects and ameliorate the action of the oxidizer on the anagenic hair causing the anagenic hair to be brittle, unruly and subject to breakage. Additionally the one or more substances can act as carriers of dye actives to deliver the dye actives to the anagenic hair surfaces. One or more of the categories of fatty substances may be combined with the oxidative hair coloring composition. Typically at least some of the categories of the fatty substances may be present in the oxidizer composition of the kit as long as they are not reactive with the oxidizer and all may be present in the tint composition.

In one embodiment of the invention, the fatty alcohol preferably may be a saturated or unsaturated linear or branched C8 to C36 fatty alcohol, preferably saturated and preferably linear, and preferably a C12 to C 20 linear saturated mono alcohol, and more preferably selected from the group consisting of cetyl alcohol, stearyl alcohol, cetearyl alcohol (mixture of cetyl and stearyl alcohols) or behenyl alcohols or mixtures thereof.

In another embodiment of the invention, the fatty carboxylic acid may be a saturated or unsaturated linear or branched C8 to C36 fatty carboxylic acid, preferably saturated and preferably linear, and preferably a C12 to C 20 linear saturated alkanoic acid, and more preferably selected from the group consisting of lauric acid. myristic acid, palmitic acid, stearic acid, arachidonic acid, palmitoleic acid, sapienic acid, oleic acid or vaccenic acid, preferably palmitic acid, stearic acid, palmitoleic acid or oleic acid, more preferably oleic acid or mixtures thereof.

In a further embodiment of the invention the fatty carboxylic ester may be a fatty carboxylic acid as described above esterified with a C2 to C6 mono, di or tri alcohol such as ethanol, isopropanol, ethylene or propylene glycol or glycerin. Preferably, the ester is oleic acid, stearic acid, palmitic acid or palmitoleic acid esterified with ethylene glycol or glycerin as a single or double ester.
In yet another embodiment of the invention, the fatty hydrocarbon may be a C16 to C36 linear or branched, unsaturated or saturated hydrocarbon. Preferably, the fatty hydrocarbon may be undecane, dodecane, tridecane, paraffin, isoparaffin, isohexadecane, isododecane, isodecane, a linear or branched hydrocarbon of mineral, animal or synthetic origin with more than 16 carbon atoms, such as a volatile or non-volatile liquid paraffin, petroleum jelly, liquid petroleum jelly, polydecene, hydrogenated polyisobutene such as Parleam^{®} or squalane or squalene.

According to the present invention, oxidative hair coloring composition may include none of the fatty substances or may include one or more wherein the concentration of each may range from about 0.1 wt% to about 20 wt%, preferably from about 0.2 wt% to about 15 wt%, more preferably from about 0.5 wt% to about 12 wt% relative to the total weight of the composition. The total concentration of all fatty substances in the oxidative hair coloring composition may range from about 1 wt% to about 50 wt%, preferably from about 1 wt% to about 25 wt% relative to the total weight of the composition.

### Component (L)

The chelant or chelator, component L, includes any cosmetically acceptable organic or inorganic compound that is capable of complexing metal ions. Included are a single chelator as well as mixtures of chelators. The one or more chelators typically are included in the oxidative hair coloring composition and in both of the tint and oxidizer compositions of the kit.

Chelants or chelators useful for the oxidative hair coloring composition for inclusion in both of the tint and oxidizer compositions as well as in the oxidative hair coloring composition include at least one of ethylenediamine tetraacetic acid (EDTA), ethylenediamine-N,N'-disuccinic acid (EDDS), diethylenetriamine pentamethylene phosphonate (DTPMP), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N-N'-bis(ortho-hydroxyphenyl acetic acid) (EDDHA), methylglycinediacetic acid trisodium salt (MGDA), 1-hydroxyethylidene-1,1-diphosphonic acid (HEDP), dimethyl glucamine (DMG), diethylenetriamine penta(methylene phosphonic acid (DTPMP) and etidronic acid (HEDP) as well as the salt or salts thereof, a derivative or derivatives thereof, and/or any combination thereof. Preferred chelators for inclusion in the tint, oxidizer and oxidative hair coloring compositions include EDTA, EDDS and HEDP. In one embodiment of the presently claimed invention, the chelant is EDDS, more preferably is (S,S)-EDDS and their salts thereof.

The concentration of the chelator or chelant in the oxidative hair coloring composition may range about 0.20 wt.% to about 1.5 wt.%, based on the total weight of the oxidative hair coloring composition. The concentrations of chelator in the tint and oxidizer compositions of the kit for preparing the oxidative hair coloring composition may be back calculated from the concentration for the oxidative hair coloring composition.

### Component M

The oxidative hair coloring compositions of the present invention preferably include a thickener, component M, in particular a polymeric thickener in an amount that is sufficient to impart a viscosity to the composition that allows for its ready application to hair without unduly dripping off the hair, as is known in the art. Typically, such an amount will be at least about 0.1 wt.%, in some embodiments, at least about 0.5 wt.%, in other embodiments, at least about 1.0 wt.%, based on the total weight of the oxidative hair coloring composition.

Exemplary thickeners for use with the present invention are organic thickeners, including but not limited to: xanthan, guar, hydroxypropyl guar, scleroglucan, methyl cellulose, ethyl cellulose (available as AQUACOTE^{®}, hydroxyethyl cellulose (NATROSOL^{®}), carboxymethyl cellulose, hydroxypropylmethyl cellulose, microcrystalline cellulose, hydroxybutylmethyl cellulose, hydroxypropyl cellulose (available as KLUCEL^{®}), hydroxyethyl ethyl cellulose, cetyl hydroxyethyl cellulose (available as NATROSOL^{®} Plus 330), N-vinylpyrrolidone (available as POVIDONE^{®}), Acrylates/Ceteth-20 Itaconate Copolymer (available as STRUCTURE^{®} 3001), hydroxypropyl starch phosphate (available as STRUCTURE^{®} ZEA), polyethoxylated urethanes or polycarbamyl polyglycol ester (e.g. PEG-150/Decyl/SMDI copolymer (e.g. ACULYN^{®} 44), PEG- 150/Stearyl/SMDI copolymer (available as ACULYN^{®} 46), trihydroxystearin (available as THIXCIN^{®}), acrylates copolymer (e.g. available as ACULYN^{®} 33) or hydrophobically modified acrylate copolymers (e.g. Acrylates / Steareth-20 Methacrylate Copolymer (available as ACULYN^{®} 22), acrylates/steareth-20 methacrylate crosspolymer (available as ACULYN^{®} 88), acrylates/vinyl neodecanoate crosspolymer (available as ACULYN^{®} 38), acrylates/beheneth-25 methacrylate copolymer (available as ACULYN^{®} 28), acrylates/C 10-30 alkyl acrylate crosspolymer (available as Carbopol^{®} ETD 2020), non-ionic amphophilic polymers comprising at least one fatty chain and at least one hydrophilic unit selected from polyether urethanes comprising at least one fatty chain.

Alternative exemplary thickeners useful for the present invention include at least one mineral thickener selected from organophilic clays, fumed silicas, or mixtures thereof. The at least one mineral thickener is present in an amount ranging from about 0.1 to about 3 wt%, based on the total weight of the oxidative hair coloring composition.

In another embodiment of the present invention, the preferred thickener is an organic thickener which may be cellulose-based thickeners (hydroxyethycellulose, hydroxypropyl cellulose, carboxymethylcellulose), guar gum and derivatives thereof (hydroxypropyl guar), gums of microbial origin (xanthan gum, scleroglucan gum), crosslinked homopolymers of acrylic acid or of acrylamido-propanesulfonic acid, and cellulose-based thickeners, such as hydroxyethylcellulose. The preferred organic thickener may be present in an amount ranging from about 0.1 to about 20 wt%, preferably from about 0.1 to about 5 wt% based on the total weight of the oxidative hair coloring composition.

### Component (N)

Optionally, embodiments of the oxidative hair coloring composition may include a conditioner for softening and at least in part protecting the hair from untoward effects of oxidation. The conditioner component N may be a cationic surfactant which may also function as a cationic surfactant for solubilizing components of the oxidative hair coloring composition. Embodiments of the conditioning component N may be selected from quaternary ammonium and tertiary amine compounds, amido ammonium and tertiary amine compounds, mono, di and polysaccharides with quaternary ammonium or tertiary amine groups, or any mixture thereof.

Non-limiting classes of these cationic surfactants include mono or di fatty alkyl or alkenyl quaternary ammonium and tertiary amine compounds, mono or di polyol quaternary ammonium and tertiary amine compounds, mono or di fatty alkyl or alkenyl benzyl quaternary ammonium and tertiary amine compounds, fatty alkyl or alkenyl aryl, polyol quaternary ammonium and tertiary amine compounds, polyol mono, di or tri saccharide alkyl quaternary ammonium and tertiary amine compounds, tertiary or quaternized aminoalkyl fatty alkyl or alkenyl ester or amide compounds, phenoxy-alkyl fatty alkyl or alkenyl quaternary ammonium and tertiary amine compounds, hydrolyzed starch with terminally quaternized ammonium and tertiary amine compounds, multi-polyol cellulose with terminal quaternized ammonium and tertiary amine compounds, sucrose, lactose mono and disaccharides, arabic, ghatti, guaicum, guar, karaya, locust bean and xanthan gums derivatized with terminal quaternized ammonium and tertiary amine compounds, and similar hydrophobic tail with cationic head organic compounds. The classes of cationic surfactants with tertiary amine groups may be protonated by the media or by organic acid to form cationic groups.

Examples of these cationic surfactants may be selected from and/or include cetrimonium chloride, steartrimonium chloride, behentrimonium chloride, behentrimonium methosulfate, behenamidopropyltrimonium methosulfate, stearamidopropyltrimonium chloride, arachidtrimonium chloride, distearyldimonium chloride, dicetyldimonium chloride, tricetylmonium chloride, oleamidopropyl dimethylamine, linoleamidopropyl dimethylamine isostearamidopropyl dimethylamine, oleyl hydroxyethyl imidazoline, stearamidopropyldimethylamine, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine, arachidamidoethyidimethylamine, benzalkonium chloride, benzodidecinium bromide, cetalkonium chloride, dicedyldimenthlammonium chloride dimethyldioctadecylamminum chloride, dioleoyl-3-trimethyl ammonium propane, lauryl methyl gluceth-10 hydroxylpropyl dimonium chloride N-oleyl-1,3-propane diamine, stearalkonium chloride and mixtures thereof. Additional cationic surfactants include cetyl trimethyl ammonium chloride available, for example, with trade name CA-2350 from Nikko Chemicals and CTAC 30KC available from KCI, stearyl trimethyl ammonium chloride with trade name Arquad 18/50 available from Akzo Nobel, hydrogenated tallow alkyl trimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methyl) pyridinium chloride; hydrophilically substituted cationic surfactants having the following INCI designations: quaternium-16, quaternium-26, quaternium-27, quaternium-30, quaternium-33, quaternium-43, quaternium-52, quaternium-53, quaternium-56, quaternium-60, quaternium-61, quaternium-62, quaternium-70, quaternium-71, quaternium-72, quaternium-75, quaternium-76 hydrolyzed collagen, quaternium-77, quaternium-78, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, and quaternium-79 hydrolyzed wheat protein, quaternium-80, quaternium-81, quaternium-82, quaternium-83, quaternium-84, and any combination thereof.

### Additional components

The oxidative hair dye composition according to the invention may further include typical and usual components such as humectants, emulsifiers, pH control agents, antioxidation agents and sunscreen/whitening agents. These components are known in the art for use with oxidative dye developments.

The humectants/emulsifiers typically are water soluble hydroxylic organic compounds with low vapor pressure and tend adhere to keratin fibers through hydrogen bonding so that at least in part they may remain on the anagenic hair after rinsing away the oxidative hair dye composition. Exemplary humectant/emulsifiers include a polyhydric, trihydric and/or dihydric alcohol (i.e., a per-hydroxy alkane, a triol or a diol) or a dimer, trimer tetramer, pentamer thereof. The polyol may be liquid or solid. The polyhydric alcohol may be a C4-C8 alkane with each carbon substituted by a hydroxyl group or a dimer or trimer thereof. The trihydric alcohol may be a C3 to C8 alkane substituted by three hydroxyl groups or a dimer or trimer thereof. The dihydric alcohol may be a C2 to C8 alkane substituted by two hydroxyl groups or a dimer or trimer thereof. Preferably, the trihydric and dihydric alcohols in pure form and without dilution with water display liquid and/or flowable properties at ambient conditions.

Exemplary polyhydric alcohols include sugar alcohols such as but not limited to erythritol, xylitol and sorbitol. Exemplary triols may be selected from propylene triol (glycerin), 1,2,3-butylene triol, 1,2,5-pentylene triol and/or 1,3,6-hexylene triol and any combination thereof. Exemplary diols may be selected from ethylene glycol, 1,2- propylene diol, 1,3 propane diol, 1,2- and 1,3-butane diol, 1,2 pentane diol, diethylene glycol, dipropylene glycol and any combination thereof. Preferred polyols include sorbitol, glycerin (propyl triol), 1,2,3-butanetriol, ethylene glycol diethylene glycol, propylene glycol, lanolin alcohol, hexylene glycol, dipropylene glycol and any combination thereof. A more preferred polyol is lanolin alcohol, glycerin, 1,2 propylene diol, 1,3 propylene diol and any combination thereof.

The agents for pH control may be added to the tint and oxidizer compositions that may be combined to form the oxidative hair coloring composition. The pH control agents manage the pH of the tint and oxidizer composition so as to provide a basic pH for the tint composition and an acidic pH for the oxidizer composition. When the tint and oxidizer compositions are combined to form the oxidative hair coloring composition, the pH may need rebalancing to a pH of from slightly above 7 to 9.5 to 10. Establishment of a basic pH of the oxidative hair coloring composition facilitates the oxidative function of the oxidizer while establishment of an acidic pH of the oxidizer composition prevents facile decomposition of the hydrogen peroxide or other peroxide producing compound. Agents for pH control include sodium hydroxide, citric acid, etidronic acid and combinations thereof.

Antioxidant inclusion with the tint composition used to form the oxidative hair coloring composition according to the invention prevents spurious oxidation of the precursor and coupler compounds of the tint while the tint composition is in package form, is on the shelf and before its use with the oxidizer composition. Useful antioxidants for this purpose include ascorbic acid, ascorbic acid stereoisomers, sodium sulfite, vitamin E and combinations thereof.

Inclusion of sunscreen agents provides protection of the colored hair from bleaching by the sun. Agents such as mica, titanium oxide and similar light reflecting materials or light absorbing materials such as benzophenone-4 and/or ethylhexyl methoxycinnamate may be included for this purpose.

Additionally, embodiments of the present invention may include penetrants; fragrances; dispersants; film-forming agents; ceramides; preserving agents; and opacifiers in the oxidative hair coloring composition.

### Exemplary Embodiments

In one embodiment of the presently claimed invention, the oxidative hair coloring composition of the invention is obtained by mixing the tint composition and oxidizer composition. The tint composition comprises preferably at least one alkalizing agent (I) and a mixture of oxidative primary dyes, for example components (A,) (B), and (C) and optionally components D, E, F and G. The oxidizer composition comprises the hydrogen peroxide (component H). Packaging an oxidizing hair coloring composition as such a two component system of tint composition and oxidizer composition is commonly used in the art and each component is formulated so that the resulting mixture is an oxidative hair coloring composition according to the invention.

An embodiment of the tint and oxidizer compositions may be formulated with appropriate carrier ingredients herein termed chassis components to deliver a composite set of carrier ingredients for the oxidative hair coloring composition that will facilitate and promote oxidative reaction of the precursor and coupler components, opening of the anagenic hair cuticles and enabling transport of the reactive agents to the cores of the anagenic hair strands.

A first exemplary chassis construction for this purpose may be designed in a cosmetically acceptable aqueous or aqueous-organic medium with a phosphate anionic surfactant of dicetyl phosphate and ceteth-10 phosphate preferably combined as Crodafos, cetearyl alcohol, glyceryl stearate, glycol distearate, optional oleic acid, glycerin and propylene glycol, monoethanolamine (MEA), ascorbic acid, EDDS-3 Na, sodium hydroxide and optional oleic acid, optional xanthan gum, optional squalane and optional behentrimonium chloride. These chassis components may be combined with the tint and oxidizer compositions as appropriate considering the chemical oxidizing potential and the required acidic pH of the oxidizer composition.

Another exemplary chassis construction for this purpose may add a nonionic surfactant such as steareth-2 to 200 with the Crodafos of the foregoing first exemplary chassis construction.

Still another exemplary chassis construction for this purpose substitutes laureth sulfate anionic surfactant and steareth-2 to 200, pareth-3 to 9 or Brij S200 for the Crodafos of the foregoing first exemplary chassis construction.

An alternative exemplary chassis construction with a cosmetically acceptable aqueous medium for this purpose may be designed with a foundation of cetearyl alcohol, ammonium hydroxide and/or MEA, glycol distearate, citric acid, propylene glycol, ascorbic acid, EDTA and optional dimethicone. To this foundation may be added one or more nonionic and/or cationic surfactants such as trideceth-2 carboxamide MEA, ceteareth-12, hexadimethrine chloride, cocoglucoside, PEG40 hydrogenated castor oil, 2-oleamido-1,3-octadecanediol, laureth-12, oleth-30, tridecth-6, lauric acid, behentrimonium chloride, carbomer. Typically, a combination of the nonionic and cationic surfactants may be combined to advantage with this chassis foundation. These surfactant combinations may include hexamethrine chloride as a cationic with any of the polyoxyethylene alkyl ethers such as laureth-12, oleth-30, ceteareth-25 nonionics or the glucoside nonionics such as cocoglucoside.

A further exemplary chassis construction has been found to enable at least substantially uniform root to tip coverage of the oxidative hair coloring composition according to the invention. This chassis construction includes a combination of dicetyl phosphate, ceteth-10 phosphate and cetearyl alcohol as the phosphate surfactant, steareth-200 as the nonionic surfactant, and xanthan gum as the viscosity control. This exemplary chassis construction does not contain fatty acid ester or fatty acid or a cationic surfactant conditioner. An alkalizer combination, a chelant combination and an organic solvent with water complete this highly preferred chassis construction.

It is believed that this highly preferred chassis construction is adapted with the EAOP coupler along with optional supplemental couplers Aminol, the aminophenols and MPD and the precursor dye compounds including MeOMe PPD and NN-bis (HOET)PPD, to deliver responsive coloration to hair strands from root to tip. In contrast, it is believed that chassis constructions based on nonionic surfactants alone, sulfate or carboxylate anionic surfactants alone or phosphate anionic surfactants alone may not be fully responsive to the differing coloration demands of the roots and tips of hair strands. It is believed that the mixture of two phosphate surfactants in combination with their fatty alcohol antecedent, the nonionic surfactant and the viscosity control compound without inclusion of a fatty acid and fatty acid ester at least in part facilitates the responsive coloration from roots to tips.

### Packaging

In one embodiment of the presently claimed invention, before use, the tint and oxidizer compositions used in the invention are normally packaged separately from one another. The compositions may be packaged in separate primary packages such as plastic bottle, sachet or tube. The components, in particular each component of a two-component composition, may however be packaged separately but within a common secondary package such as a carton or in different compartment of an aerosol or foam bottle, as in known in the trade. A conditioning composition, which can be applied after rinsing of the oxidative hair coloring composition, may also be packaged in such secondary package. On the other hand, the different components of the invention such as the third component, may be sold separately from the other components.

### Method of Hair Dying

Application of the oxidative hair coloring composition to the hair may be undertaken in several ways. Application of the oxidative hair coloring composition may take place on the whole head of hair of an end user. As used herein, the "whole head of hair" means that the hair all over the head from the root of the hair to the tip of the hair is included in the application process. By contrast, the application of the oxidative hair coloring composition may take place only on the root portion of the hair. The application to the root portion of the hair may still be over the entire head of the end user, but application of the oxidative hair coloring composition is applied only to the section of hair closest to the head (root portion), which is between about 0.01 mm to about 40 mm from the scalp of the head. After application to the root portion, product may be applied to the rest of the hair at a later stage to prevent over processing of the hair in the lengths and ends. Also, application may take place on a portion of hair. Application of a portion of hair is commonly referred to as highlighting or lowlighting. The portion of hair may be physically separated from the whole head of hair in a hair bundle or may be a smaller portion of hair than the whole head of hair. A hair bundle may be physically separated from a whole head of hair by a device including a plastic cap through which hair bundles are formed when hair is pulled through orifices in the plastic cap, metal foils encompassing a hair bundle, strand separators applied to hair at the root portion, or similar devices.

When present, an optional conditioning agent can be provided in an additional container. In the latter case, the conditioner can be mixed immediately before use and applied together with the other components, or the content of the additional container can be applied (after an optional rinse step) as a post-treatment immediately after the oxidative hair coloring composition.

According to one method for oxidatively coloring hair, the method comprises mixing a tint composition and an oxidizer composition and optionally a third component comprising a second non-ammonia alkalizing agent together to form the oxidative hair coloring composition, applying the oxidative hair coloring composition to the hair to form a treated hair surface, waiting for a period of 5-50 minutes, such as 20-35 minutes, and then removing the hair coloring composition from the treated hair surface through rinsing with water.

The methods of coloring hair also may further comprise working the oxidative hair coloring composition into the treated hair surface by hand or by a tool for a few minutes to ensure uniform application to the entire treated hair surface. The oxidative hair coloring composition remains on the treated hair surface while the end hair color develops for a time period of 5 to 50 minutes to form oxidatively colored hair. The consumer then rinses his/her oxidatively colored hair thoroughly with tap water and allows it to dry and/or styles the oxidatively colored hair.

In one embodiment of the presently claimed invention, a method of treating hair with the oxidative hair coloring composition preferably comprises the steps of:
a. providing a tint composition as described herein;
b. providing an oxidizer composition as described herein;
c. mixing the oxidizing composition and the tint composition to obtain a mixed oxidative hair coloring composition as described herein;
d. applying the mixed oxidative hair coloring composition for the oxidative dyeing of keratin fibres onto the hair;
e. leaving the composition on the hair for 5 to 50 minutes; and
f. subsequently rinsing the oxidative hair coloring composition from the hair.

In one embodiment of the presently claimed invention, the oxidative hair coloring composition may be obtained by mixing immediately prior to use a tint composition and the oxidizing composition. A sufficient amount of the mixed oxidative hair coloring composition is applied to the hair, according to the hair abundance, generally from 20 to 250 grams depending on the amount of hair to be colored. Upon such preparation, the oxidative hair coloring composition is applied to the hair to be dyed and remains in contact with the hair for an amount of time effective to dye the hair. Typically, the oxidative hair coloring composition is allowed to act on the hair from 5 to 50, preferably 10 to 40, preferably 20 to 35 minutes, at a temperature ranging from 15 to 50 °C. Thereafter, the hair is rinsed with water to remove the oxidative hair coloring composition and dried. If necessary, the hair is washed with a shampoo and rinsed, e.g., with water or a weakly acidic solution, such as a citric acid or tartaric acid solution, and dried. Optionally, a separate conditioning product may also be provided.

The oxidative hair coloring composition can be applied on hair via an applicator bottle or brush. It can be used on full head or partly on single strands (highlight application) as common highlight applicator foils, caps and special applicators can be used, but also freehand techniques such as balayage, with brush and/or combs can be possible. The oxidative hair coloring composition can also be applied as a mousse via a manual spray, a pressurized container or an aerosol mousse. The oxidative hair coloring composition may be dispensed as a solid form to which water is added to generate the oxidant and form a thickened vehicle suitable for hair coloring.

### EXAMPLE

The following example illustrates the formulations and performance results of oxidative hair coloring composition according to the present invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

### Hair tresses used for testing.

The tresses may be subjected to the following treatment to reflect consumers lengths and end hair. Natural white undamaged human hair is purchased (Kerling International Haarfabrik GmbH, Backnang, Germany) in the form of 10 cm long and 1 cm wide tresses. The tress may be treated with a mixture of Blondor Multi-Blonde bleach powder available from Wella Professionals mixed 1 part with 1.5 parts of 12% Welloxon Perfect available from Wella Professionals. About 4 g of this mixture may be applied to each gram of hair. The tresses may be then incubated in an oven at 45 °C for 30 minutes after which they may be rinsed in water, 37 +-3 °C with a flow rate of 4 L / min for 2 minutes and the hair may be then dried with a standard Hair dryer from Wella.

### Method of applying hair coloring formulation to hair

All products and equipment for the hair dye-out may be equilibrated to 30 °C in a circulation oven overnight. Equal weights (approximately 4 g each) of tint composition and oxidizing composition may be mixed in a plastic bowl using a dye-out brush. This mixture is referred to as the oxidative hair coloring composition for the composition of the invention and as the resorcinol coloring composition for the comparative composition.

The hair tress may be placed on a plastic plate and about 2g of coloring composition or resorcinol composition may be applied to the free hair portion of a tress and the color developed at 30°C for 30 minutes in a 30°C air circulation oven. After color development and removal from the oven, excess coloring composition may be squeezed out from the tress by hand, wearing nitrile gloves and the tress washed in 4 L min water at 37 C for 2 min. The hair may be then blow dried.

The L* value (lightness value), a* (green to red component value) and b* (blue to yellow component value) of hair swatches colored with each of the hair coloring composition C1, C2 and C3 may be measured using a Minolta 2600d spectrophotometer.

For each tress ten measurements may be taken, five on each side of the tress. The error analysis may be done using standard statistical formulae and functions in Microsoft Excel.

The formulations of the oxidative hair coloring composition and the comparative resorcinol composition may be prepared according to the following methods:
A chassis premix formula that may be use for the tint formulation and the oxidizing formulation. Components of the chassis premix formula that would undergo oxidation and that are incompatible with the oxidizing formulation are to be omitted when used as a chassis for the oxidizing formulation.

The chassis premix formula may include preferred tint components such as but not limited to one or more of phosphate surfactant, nonionic surfactant, fatty alcohol, fatty esters such as glyceryl stearate and glycol distearate, fatty acids such as palmitoleic, oleic or linoleic acids, solvents such as glycerin and propylene glycol, alkalizers such as monoethanolamine (MEA), 2-aminopropanol, ammonium hydroxide, ammonium carbonate, sodium hydroxide, ascorbic acid, chelators such as EDDS-3 Na, DTPMP, conditioners such as fatty quaternary ammonium cationic surfactants and thickeners such as xanthan gum and/or guar gum. This premix formula may be used for the tint formulation of the oxidative hair coloring composition and the comparative resorcinol tint composition. The alkalizers, ascorbic acid, conditioners and thickeners of the premix formula may be omitted when used with the oxidizing formulation and citric acid may be included.

The tint composition for the oxidative hair coloring composition included 2-methoxymethyl-p-phenylene diamine, NN-bis(hydroxyethyl)-PPD sulfate, p-amino phenol, 2-amino-5-ethyl phenol (E-OAP), hydroxyethyl-3,4-methylenedioxyanaline (Aminol), m-aminophenol, 2,4-diaminophenoxyethanol and 2-methyl-5-hydroxyethylaminophenol. The concentrations of these precursor and coupler compounds are provided in the table.

The tint composition for the comparative resorcinol composition included 2-methoxymethyl-p-phenylene diamine, NN-bis(w-hydroxyethyl)-PPD sulfate, resorcinol, m-aminophenol and 1-naphthol. The concentrations of these compounds are provided in the table.

Pursuant to the hair swatch color treatment procedure described above, hair swatches may be prepared with the oxidative hair coloring composition and the comparative resorcinol composition. The colors of the resulting hair swatches may be examined by the Minolta procedure give above to determine the L*a*b* color space provided by the oxidative hair coloring composition and the comparative resorcinol composition. These results are provided on the following table. These results demonstrate that the oxidative hair coloring composition delivers a color space the same as the comparative resorcinol composition but the oxidative hair coloring composition avoids the environmental, health and toxic concerns of resorcinol.

### Resorcinol and Et-OAP Tint Comparison 1

| | **Shade Number and Name** | | **Resorcinol shade#4** | | **Shade#4 EtOAP** |
|---|---|---|---|---|---|
| | | | Wt% relative to total composition weight | | Wt% relative to total composition weight |
| | **Primaries** | | | | |
| | 2-METHOXYMETHYL-P-PHENYLENEDIAMINE | | 2.0500 | | **0.9540** |
| | N,N-BIS(2-HYDROXYETHYL)-PPD SULFATE* | | 0.2000 | | 0.2000 |
| | P-Aminophenol | | | | 0.0500 |

| | **Couplers** | | | | |
|---|---|---|---|---|---|
| | Resorcinol | | 0.9000 | | |
| | Et-OAP/Ethyloxygelb * * | | | | **0.6000** |
| | M-Aminophenol | | 0.1600 | | 0.1500 |
| | Aminol | | | | 0.2700 |
| | 2,4-DIAMINOPHENOXYETHANOL HCL | | | | 0.0100 |
| | 2-METHYL-5-HYDROXYETHYLAMINOPHENOL | | | | 0.0010 |
| | NAP/1-Naphthol | | 0.0500 | | |
| | Orange PMP | | | | 0.1000 |
| | Cassis components# | | QED 100% | | QED 100% |

**Minolta**
**Color**
**Reading**

| | | | | | | | |
|---|---|---|---|---|---|---|---|
| | Natural White hair | | L*(D65) | | a*(D65) | | b*(D65) |
| | Current NNE shade#4 | | 22.55 | | 3.75 | | 6.09 |
| | NNE shade#4 with EtOAP | | 22.6 | | 1.85 | | 6.24 |
| | | | | | | | |
| | Level 3 - 50% Blended Grey hair | | L*(D65) | | a*(D65) | | b*(D65) |
| | Current Resorcinol shade#4 | | 18.31 | | 2.17 | | 4.45 |
| | Shade#4 with EtOAP | | 18.63 | | 3.22 | | 4.57 |

### Resorcinol and Et-OAP Tint Comparison 2

| NNE Shade No. 5G-Medium Golden Brown | | | | | |
|---|---|---|---|---|---|
| | Shade Number and Name | | Current NNE Golden Brown No 5G Resorcinol | | New NNE Golden Brown No 5G E-OAP |
| | | | wt% relative to 100% total composition weight | | wt% relative to 100% total composition weight |
| | Ratio of Coupler to Primary (theoretical 2:1) | | 1.91: 1 | | 1.08:1 |
| | Primaries | | | | |
| | 2-Methoxymethyl-p-phenyldiamine | | 1.38 | | 0.52 |
| | N, N-bis(2-hydroxyethyl)-PPD sulfate* | | 0.016 | | 0.05 |
| | p-aminophenol | | 0.17 | | 0.22 |
| Couplers | | | | | |
| | resorcinol | | 0.4 | | |
| | Et-OAP/ethyloxygelb** | | 0.012 | | 0.4 |
| | M-aminophenol | | 0.24 | | 0.15 |
| | aminol | | | | 0.06 |
| | 4-amino-2-hydroxytoluene | | 0.43 | | 0 |
| | 2-methyl-5-hydroxyethylaminophenol | | | | 0.03 |
| | 2-methylresorcinol | | 0.52 | | |
| | Orange PMP*** | | | | 0.1 |
| Chassis components^{#} | | | QED 100% | | QED 100% |

### Minolta Color Reading

| | Natural White Hair | | L*(D65) | | a*(D65) | | b*(D65) |
|---|---|---|---|---|---|---|---|
| | Current NNE Formulation | | 30.54 | | 7.75 | | 14.4 |
| | New NNE Formulation with Et-OAP | | 29.8 | | 7.69 | | 14.9 |
| | | | | | | | |

| | Level-3 50% Blended Grey Hair | | L*(D65) | | a*(D65) | | b*(D65) |
|---|---|---|---|---|---|---|---|
| | Current NNE Formulation | | 23.27 | | 6.46 | | 9.78 |
| | New NNE Formulation with Et-OAP | | 23.52 | | 5.81 | | 9.37 |

| | | | | | | | |
|---|---|---|---|---|---|---|---|
| Legend * PPD is paraphenylene diamine **Et-OAP is 2-amino-5-ethylphenol, a.k.a p-ethyl-o-amino phenol, ethoxygelb is ethoxy yellow ***PMP is 1-phenyl-3-methyl-5-pyrazolone # Chassis components include but are not limited to surfactants such as nonionics and/or anionics (carboxylates and/or sulfonates and/or organophosphates), alkalizers, antioxidants, chelators, viscosity control agents, conditioners, emolliants, fatty acids, fatty alcohols, fatty esters, water and C2-C6 mono and di alcohols. | | | | | | | |

The highly preferred chassis for the tint composition part of the oxidative hair coloration composition includes about 0.2 wt% to about 25 wt%, preferably about 0.2 wt% to about 20 wt% of a mixture of dicetyl phosphate and ceteth-10 phosphate and cetearyl alcohol at a wt% ratio of about 1 to about 15 wt%, preferably about 1.5 to about 10 wt% of each of the two phosphates and a remainder of cetearyl alcohol relative to the total weight of the mixture; xanthan gum at about 0.1 wt% to about 0.2 wt%, steareth-200 at about 0.2 wt% to about 0.5 wt% with the alkalizers ammonium hydroxide, aminopropanol, ethanolamine and ammonium carbonate, EDDS and DTPMP and propylene glycol/water.

### MISCELLANEOUS STATEMENTS

The invention has been described broadly and generically herein. Each of the narrower species and subgeneric groupings falling within the generic disclosure also form part of the invention. This includes the generic description of the invention with a proviso or negative limitation removing any matter from the genus, regardless of whether or not the excised material is specifically recited herein. The inventions, examples, results and statement of embodiments described, stated and claimed herein may have attributes and embodiments include, but not limited to, those set forth or described or referenced in this application.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed and as provided by the statements of embodiments. Thus, it will be understood that although the present invention has been specifically disclosed by various nonlimiting embodiments and/or preferred nonlimiting embodiments and optional features, any and all modifications and variations of the concepts herein disclosed that may be resorted to by those skilled in the art are considered to be within the scope of this invention as defined by the appended claims and the statements of embodiments.

All patents, publications, scientific articles, web sites and other documents and ministerial references or mentioned herein are indicative of the levels of skill of those skilled in the art to which the invention pertains, and each such referenced document and material is hereby incorporated by reference to the same extent as if it had been incorporated verbatim and set forth in its entirety herein. The right is reserved to physically incorporate into this specification any and all materials and information from any such patent, publication, scientific article, web site, electronically available information, textbook or other referenced material or document.

The written description of this patent application includes all claims, examples and statements of embodiments. All claims and statements of embodiments including all original claims are hereby incorporated by reference in their entirety into the written description portion of the specification and the right is reserved to physically incorporate into the written description or any other portion of the application any and all such claims and statements of embodiments. Thus, for example, under no circumstances may the patent be interpreted as allegedly not providing a written description for a claim on the assertion that the precise wording of the claim is not set forth in haec verba in written description portion of the patent.

While the invention has been described in conjunction with the detailed description thereof, the foregoing description is intended to illustrate and not limit the scope of the invention, which is defined by the scope of the appended claims and the statements of embodiments. Thus, from the foregoing, it will be appreciated that, although specific nonlimiting embodiments of the invention have been described herein for the purpose of illustration, various modifications may be made without deviating from the scope of the invention. Other aspects, advantages, and modifications are within the scope of the following claims and the present invention is not limited except as by the appended claims and the statements of embodiments.

The specific methods and compositions described herein are representative of preferred nonlimiting embodiments and are exemplary and not intended as limitations on the scope of the invention. Other objects, aspects, and embodiments will occur to those skilled in the art upon consideration of this specification and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. Thus, for example, in each instance herein, in nonlimiting embodiments or examples of the present invention, the terms "comprising", "including", "containing", "providing" and the like are to be read expansively and without limitation.

The methods and processes illustratively described herein suitably may be practiced in differing orders of steps, and that they are not necessarily restricted to the orders of steps indicated herein or in the claims.

## Claims

1. An oxidative hair coloring composition comprising a cosmetically acceptable medium and at least components A, B and C wherein:
component A) is at least a first oxidative coupler comprising 2-amino-5-ethylphenol of Formula I, its addition salts, its solvates and/or solvates of its salts, 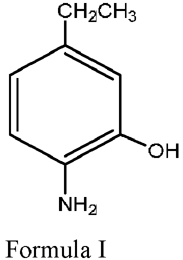
component B) is at least a second oxidative coupler comprising hydroxylethyl-3,4-methylenedioxyaniline of Formula II, its addition salts, it solvate and/or solvates of its salts, 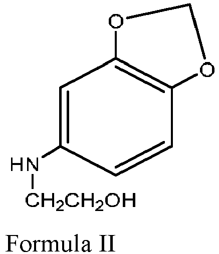
component C) is at least a first oxidative primary dye precursor comprising 2-methoxymethyl-p-phenylene diamine of Formula III, its addition salts, its solvates and/or solvates of its salts,

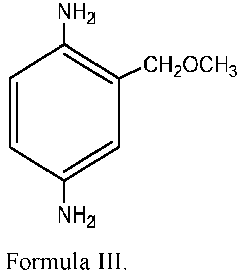

2. The oxidative hair coloring composition according to claim 1 further comprising one or more additional oxidative dye precursors as component D and one or more oxidative couplers as component E wherein:
component D comprises at least one or more of:
i) N,N-bis(2-hydroxyethyl-p-phenylenediamine, its addition salts, its solvates and/or solvates of is salts, and/or
ii) p-aminophenol, its addition salts, its solvates and/or solvates of is salts, and/or
iii) any combination Di) and Dii);
component E comprises at least one or more of:
i) 2,4-diaminophenoxyethanol, its addition salts, its solvates and/or solvates of its salts; and/or,
ii) m-aminophenol its addition salts, its solvates and/or solvates of its salts; and/or,
iii) 2-methyl-5-hydroxyethylaminophenol its addition salts, its solvates and/or solvates of its salts; and/or,
iv) 1-naphthol and its solvates; and/or,
v) 1-phenyl-3-methyl-5-pyrazolone, its addition salts, its solvates and/or solvates of its salts; and/or,
vi) any combination of Ei), ii), iii) iv) and v); and/or
any combination of components Di), ii), iii), Ei), ii), iii), iv), v) and vi).

3. The oxidative hair coloring composition according to any of the preceding claims wherein the composition further comprises one or more of components F and G and substitute C and any combination thereof wherein:
component F is at least one additional oxidative coupler comprising m-aminophenol, 4-amino-2-hydroxytoluene, 6-hydroxybenzomorpholine, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 2-amino-5-ethylphenol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotoluene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindoline, phenyl methyl pyrazolone, 5-((2-hydroxyethyl)amino)-1,3-benzodioxol, hydroquinone, 4-formyl-1-methylquinolinium-p-toluenesulfonate, 2-amino-6-chloro-4-nitrophenol, 3,4-methylenedioxyphenol and addition salts, solvates and/or solvates of salts thereof, and any combination thereof;
component G is an oxidative primary dye precursor comprising at least one of hydroxyethyl-p-phenylenediamine sulphate, hydroxypropyl bis(n-hydroxyethyl-p-phenylenediamine), 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol, 2,3-diaminodihydroxypyrazolopyrazolone dimethosulfonate, 4,5-diamino-1-hexylpyrazol, 2,3-Diamino-6,7-dihydro-1H,5H-pyrazolo[1,2-a]pyrazol-1-one, hydroxypropyl-p-phenylenediamine, dimethylpiperazinium aminopyrazolopyridine chloride hydrochloride, 4,5-Diamino-3-methyl-1H-pyrazole-1-ethanol, 1-Hexyl-3-methyl-1H-pyrazole-4,5-diamine, methylimidazoliumpropyl p-phenylenediamine, hydroxyethoxy aminopyrazolopyridine, 2-(2,5-diaminophenyl)ethanol, N,N-bis-(2-hydroxyethyl)-p-phenylenediamine and addition salts, solvates and/or solvates of salts thereof, and any combination thereof; and
substitute component C wherein 2-methoxymethyl-p-phenylene diamine is replaced by 2,5-toluene diamine sulfate as substitute component C.

4. The oxidative hair coloring composition according to any of the preceding claims further comprising as component H at least one oxidizing agent, preferably comprising hydrogen peroxide, preferably present in an amount in a range of from about 1.0 wt% to about 10.0 wt% relative to the total weight of the hair coloring composition.

5. The oxidative hair coloring composition according to any of the preceding claims further comprising as component I at least one alkalizing agent comprising at least one of 2-amino-1-propanol, 2-amino-1-butanol, 2-dimethylamino-2-ethyl-1-propanol, 2-amino-2methyl propanol, 3-amino-1-propanol, ammonia, ammonium hydroxide, ammonium carbonate and/or monoethanolamine and any combination thereof, preferably present in an amount in a range of from about 0.1 wt% to about 10.0 wt% relative to the total weight of the hair coloring composition.

6. The oxidative hair coloring composition according to any of the preceding claims further comprising as component J one or more anionic and/or nonionic surfactants comprising at least one of a phosphate anionic surfactant, a sulfate anionic surfactant, a nonionic polyethoxy alkyl ether surfactant, a betaine, a polysorbate, a fatty alcohol pyrrolidone or any combination thereof, preferably present in an amount of from about 0.1 wt% to about 15 wt% relative to the total weight of the hair coloring composition.

7. The oxidative hair coloring composition according to any of the preceding claims further comprising one or more of components J, K and L wherein:
component J comprises dicetyl phosphate, ceteth-10 phosphate, laureth-2 to 200 sulfate, cocyl isethionate, lauryl sulfate, steareth-2 to 200, octadeceth-2 to 200, laureth-2 to 200, undeceth-2 to 200, pareth-2-200, cocamidopropyl betaine, polysorbate -2 to 80 polyoxyethylene and/or lauryl pyrrolidone and/or any combination thereof;
component K comprises one or more of a C8 - C20 fatty alcohol, a C8 to C20 fatty acid, a glyceryl or glycol ester of a C8 to C20 fatty acid, and/or any combination thereof.
component L comprises at least one of ethylenediamine tetraacetic acid (EDTA), ethylenediamine-N,N'-disuccinic acid (EDDS), diethylenetriamine pentamethylene phosphonate (DTPMP), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N-N'-bis(ortho-hydroxyphenyl acetic acid) (EDDHA), dimethyl glucamine (DMG), salts, derivatives, and/or any combination thereof.

8. The oxidative hair coloring composition according to any of the preceding claims, further comprising one or more additive components of a thickener, a conditioner, a humectant/emulsifier, an antioxidant, a pH control agent and/or a sunscreen, opacifying agent, sodium sulfate, xanthan gum, ethoxydiglycol, oleamide MIPA, beeswax, hydrolyzed keratin, polyquaternium-22, soytrimonium chloride, dicetydimonium chloride, glycerin, propylene glycol, lanolin alcohol, hexylene glycol dipropylene glycol, ethanol, isopropanol, citric acid, sodium hydroxide, etidronic acid, ascorbic acid sodium sulfite, vitamin E, fragrance and/or any combination thereof.

9. The oxidative hair coloring composition according to any of the preceding claims wherein the molar ratio of oxidative couplers to oxidative primary dye precursors is from 1:1 to about 1:1.5 and the concentration of each oxidative coupler and each oxidative primary dye precursor may in a weight percent range of from about 0.1 wt% to about 10 wt%, preferably 0.1 wt% to about 5 wt% with the sum of the concentrations of all precursors and couplers being from 0.5 wt % to about 10 wt%.

10. A kit comprising:
a. a tint composition comprising at least components A-C according to claim 1 and component I according to claim 5, and
b. an oxidizer composition comprising as component H at least one oxidizing agent.

11. A kit according to claim 10 further comprising addition to the tint composition of one or more of components D, E, F and G and any combination thereof of any of the preceding claims 2 and 3 and addition optionally one or more of components I, J, K, L of claims 5, 6 and 7 and additive components of claim 8.

12. A method for coloring hair with a kit according to claims 10 or 11 comprising the steps of:
a. combining the tint composition and the oxidizer composition of the kit to provide the oxidative hair coloring composition according to any of preceding claims 1-9;
b. applying the oxidative hair coloring composition of step a to the hair,
c. allowing the oxidative hair coloring composition to remain on the hair for a period of from about 5 to about 50 minutes, and
d. subsequently rinsing the oxidative hair coloring composition from the hair.

13. The oxidative hair coloration composition according to any of the preceding claims 1-3, further comprising at least a chassis comprising at least a mixture of a three component phosphate surfactant comprising cetearyl alcohol, dicetyl phosphate and ceteth-10 phosphate, a nonionic surfactant comprising steareth-200, xanthan gum, one or more one alkalizers including ammonium hydroxide, aminopropanol, ethanolamine and ammonium carbonate, EDDS and DTPHP, a glycol and water.

14. The oxidative hair coloration composition according to claim 13 in combination with component H comprising at least one oxidizing agent.

15. Use of the oxidative hair coloration composition of any of preceding claims 1-9, 13, 14 for oxidative coloration of hair.
